# EUROPEAN PATENT SPECIFICATION

(11) **EP 1 952 156 B1**
(45) Date of publication and mention of the grant of the patent: **04.05.2011**
(21) Application number: 06837479.2
(22) Date of filing: 14.11.2006
(51) Int. Cl.: G01N 33/566, G01N 33/74, G01N 33/53, G01N 33/50, G01N 33/68

(54) **DIAGNOSTIC METHOD FOR PREECLAMPSIA**
DIAGNOSEVERFAHREN FÜR PRÄEKLAMPSIE
PROCEDE POUR LE DIAGNOSTIC DE LA PRE-ECLAMPSIE

(30) Priority: 14.11.2005 US 736659 P; 03.03.2006 US 367126
(43) Date of publication of application: 06.08.2008
(73) Proprietor: Abbott Laboratories, Abbott Park, Illinois 60064-6008 (US)
(72) Inventor: SOGIN, David, C., Highland Park, Illinois 60035 (US); LAIRD, Donald, M., Mundelein, Illinois 60060 (US); YU, Zhiguang, Libertyville, Illinois 60048 (US); DOSS, Robert, C., Libertyville, Illinois 60048 (US)
(74) Representative: Modiano, Micaela Nadia
(86) International application number: PCT/US2006/044059
(87) International publication number: WO 2007/059065

(56) References cited:
- EP-A- 0 114 528
- EP-A- 1 530 047
- EP-A- 1 615 035
- WO-A-00/26674
- WO-A-98/28621
- WO-A-02/099421
- WO-A-03/043487
- WO-A-2005/077007
- HORNIG C ET AL: "Release and complex formation of soluble VEGFR-1 from endothelial cells and biological fluids" LABORATORY INVESTIGATION, UNITED STATES AND CANADIAN ACADEMY OF PATHOLOGY, BALTIMORE,, US, vol. 80, no. 4, April 2000 (2000-04), pages 443-454, XP002993167 ISSN: 0023-6837
- HORNIG C ET AL: "Detection and quantification of complexed and free soluble human vascular endothelial growth factor receptor-1 (sVEGFR-1) by ELISA" JOURNAL OF IMMUNOLOGICAL METHODS, ELSEVIER SCIENCE PUBLISHERS B.V.,AMSTERDAM, NL, vol. 226, no. 1-2, 24 June 1999 (1999-06-24), pages 169-177, XP004171370 ISSN: 0022-1759
- BELGORE FUNMI M ET AL: "MEASUREMENT OF FREE AND COMPLEXED SOLUBLE VASCULAR ENDOTHELIAL GROWTH FACTOR RECEPTOR, FLT-1, IN FLUID SAMPLES: DEVELOPMENT AND APPLICATION OF TWO NEW IMMUNOASSAYS" CLINICAL SCIENCE, BIOCHEMICAL SOCIETY AND THE MEDICAL RESEARCH SOCIETY, LONDON,, GB, vol. 100, no. 5, May 2001 (2001-05), pages 567-575, XP008076526 ISSN: 0143-5221
- AHMAD SHAKIL ET AL: "Elevated placental soluble vascular endothelial growth factor receptor-1 inhibits angiogenesis in preeclampsia" CIRCULATION RESEARCH, vol. 95, no. 9, 29 October 2004 (2004-10-29), pages 884-891, XP008078922 ISSN: 0009-7330
- LAM C ET AL: "CIRCULATING ANGIOGENIC FACTORS IN THE PATHOGENESIS AND PREDICTION OF PREECLAMPSIA" HYPERTENSION, XX, XX, vol. 46, no. 5, November 2005 (2005-11), pages 1077-1085, XP009069173 ISSN: 0194-911X
- KENDALL R L ET AL: "SPECIFICITY OF VASCULAR ENDOTHELIAL CELL GROWTH FACTOR RECEPTOR LIGAND BINDING DOMAINS" BIOCHEMICAL AND BIOPHYSICAL RESEARCH COMMUNICATIONS, ACADEMIC PRESS INC. ORLANDO, FL, US, vol. 201, no. 1, 30 May 1994 (1994-05-30), pages 326-330, XP000611910 ISSN: 0006-291X
- HIRASHIMA C ET AL: "ESTABLISHING REFERENCE VALUES FOR BOTH TOTAL SOLUBLE FMS-LIKE TYROSINE KINASE 1 AND FREE PLACENTAL GROWTH FACTOR IN PREGNANT WOMEN" HYPERTENSION RESEARCH. CLINICAL AND EXPERIMENTAL, OSAKA, JP, vol. 28, no. 9, September 2005 (2005-09), pages 727-732, XP008076525 ISSN: 0916-9636

## Description

### Technical Field of the Invention

The Field of the invention relates to the detection of placental growth factor (PIGF), soluble fms-like tyrosine kinase (sFlt-1), and related molecules in biological samples that are preferably obtained from patients.

### Background of the Invention

Immunodiagnostics enables the detection, diagnosis, prognosis of diseases, dysfunctions, and other conditions afflicting or affecting animals, including humans. It has become highly desirable to perform immunodiagnostics testing with the aid of automated testing equipment that minimizes the investigator's time handling samples and data. The rapid commercial growth of immunodiagnostics since 1980 has been made possible in part by technology permitting the rapid and efficient isolation of antibodies and/or antibody fragments that bind with sufficient specificity to markers found in biological samples, so that the marker can be recognized. Even more desirable for some immunodiagnostics testing has been the use of monoclonal antibodies, which in many instances allows the skilled artisan to carefully tailor the performance, specificity, and sensitivity of an assay to particular needs. Antibodies also tend to be predictable molecules that are somewhat amenable to improvement by genetic re-engineering. Hence, they have become essential elements of modem immunodiagnostics agents.

Other reagents are available for the detection of markers in biological samples, but the need to carefully characterize these agents and develop unique techniques for their use in immunoassays has somewhat discouraged their use in modem immunodiagnostics. This is particularly true when the non-antibody reagent is a polypeptide or protein.

VEGF and P1GF belong to a family of regulatory peptides that can control blood vessel formation and vascular permeability. These proteins are believed to interact with Flt-1 and KDR/FLK1 to achieve this function (Mattei et al., Genomics, 32, 168-169, (1996)). There are currently 3 putative isoforms of PlGF identified: PlGF1, PlGF2, and P1GF3. P1GF2 can bind with heparin. PlGF2 is believed to be capable of binding neuropilin-1 in human umbilical vein endothelial cells in a heparin-dependent fashion. Neuropilin-1 is also believed to be able to bind with P1GF1 with lower affinity (Migdal et al., J Biol Chem, 273, 22272-22278 (1998)).

P1GF is believed to be capable of stimulating angiogenesis and collateral growth in ischemic heart and limb with good efficiency (Luttun et al., Nature Med 8, 831-840 (2002)). Activation of Flt-1 by P1GF can cause angiogenesis. Both VEGF and P1GF bind to Flt-1, however, P1GF binding with Flt-1 is believed to cause different biological effects than VEGF binding to Flt-1.

In pregnant women suffering from preeclampsia, increased soluble Flt-1 (sFlt-1) may cause decreased circulating levels of free VEGF and especially P1GF, resulting in endothelial cell dysfunction that could be relieved by exogenous VEGF and P1GF (Maynard et al., J Clin Invest, 111, 649-658 (2003)). Serum levels of P1GF were significantly lower in women who later had preeclampsia, than in women who did not later develop preeclampsia, in a study reported by Levine et al. (New Eng J Med, 350, 672-683 (2004)). The study suggested that the difference might be perceptible by about 13 to about 16 weeks of gestation, and the greatest difference in P1GF levels was apparent closer to the onset of preeclampsia. Levine et al. also suggested that an increase in the total sFlt-1 level in the blood was also more pronounced in the preeclamptic women. Levine et al. therefore suggested that increased levels of total sFlt-1 and lower levels of P1GF could predict the subsequent development of preeclampsia.

sFlt-1 is believed to be an alternately spliced form of flt-1 resulting in a soluble variant of the Flt-1 protein and can bind both vascular endothelial growth factor (VEGF) with high affinity (Kendall et al., Biophys Res Commun, 226, 324-328 (1996)) and PlGF. Domain deletion studies of the sFlt-1 have shown that (s)Flt-1 domains 2 and 3 permit binding to VEGF with almost the same affinity as sFlt-1 and that domain 2 alone binds only 60-fold less tightly than the full-length sFlt-1.

WO2005/077007 mentions measurement of free PlGF and sFlt-1 in bound, total or free form and VEGF (p. 25, para. 2) but no tools for measuring free sFlt-1 are disclosed. The calculation of a ratio of sFlt-1 to VEGF and PlGF is proposed (see also last para. on page 24 to para. 1 on page 25).

### Summary of the Invention

The invention in its broadest form is defined in independent claims 1 and 2. Preferred embodiments are defined in claims 3-29.

Claims 1 and 2 read as follows:
1. A method of predicting a lower risk of preeclampsia comprising: measuring the amount of free sFlt-1 in a biological sample obtained from a pregnant woman, measuring the amount of free PlGF in the biological sample, and comparing the observed ratio to a predetermined value or range of values.
2. A method of predicting a lower risk of preeclampsia comprising: measuring the amounts of total sFlt-1 and bound sFlt-1 in a biological sample obtained from a pregnant woman, measuring the amount of free PlGF in the biological sample, and comparing the observed ratio to a predetermined value or range of values.

The disclosure involves the use of a proteinaceous binding partner, other than a portion of an antibody, used to detect the quantity or concentration of a second binding partner, other than a portion of an antibody, in a biological sample. Only one antibody or portion thereof is preferably used in the inventive method. Binding partners of the invention include, placental growth factor (P1GF) and soluble fms-like tyrosine kinase (sFIt-1), which is a portion of Flt-1 generated by alternative splicing of the Flt-1 gene product and is capable of binding with PlGF.

The disclosure also provides a method of determining the quantity of sFlt-1 that is not bound to P1GF ("free sFlt-1") and a method of determining the quantity of P1GF that is not bound to sFlt-1 ("free PlGF").

Moreover, the disclosure provides a method of determining the ratio of free sFlt-1 to free PlGF.

In another embodiment, the ratio of free sFlt-1 to free P1GF is used to diagnose, predict, monitor, or monitor therapy of preeclampsia.

### Brief Description of the Drawings

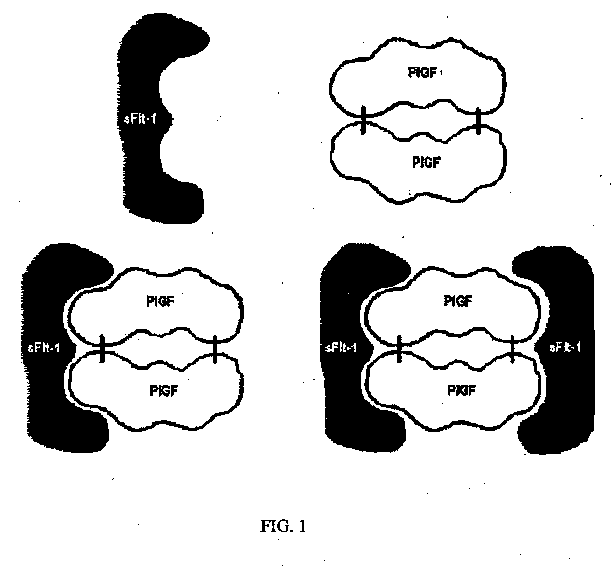

Figure 1 illustrates the ability of sFlt-1 to interact with P1GF. Although the binding of two molecules of sFlt-1 with a homodimer of PlGF has been suggested by observations of experimental systems, the inventors recognize that this state may not exist in vivo or may exist at insignificant levels, but is presented in Fig. 1 because the inventive method is useful for the detection of such complexes, if they exist.

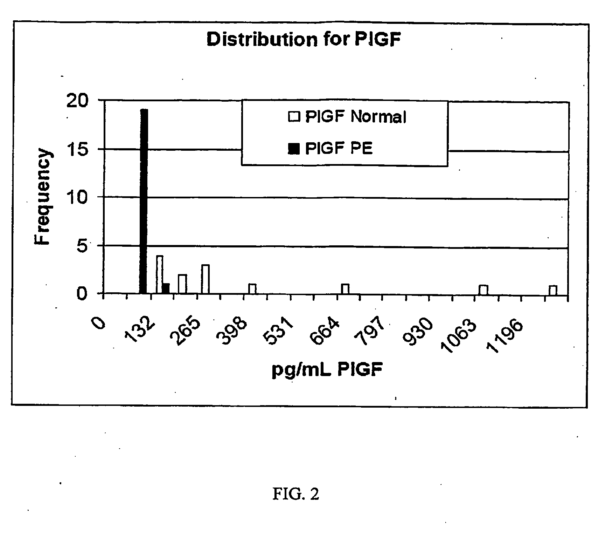

Figure 2 depicts a histogram of PlGF levels observed by an immunoassay employing a monoclonal antibody used to capture free P1GF and a polyclonal antibody used to detect PlGF in a small number of human samples collected and investigated under ethically appropriate conditions. Figure 2 demonstrates that low levels of PlGF are associated with preeclamptic pregnancies (PE), and also that the ability to separate non-preeclamptic (Normal) from preeclamptic pregnancies using two antibodies to PLGF is in need of improvement.

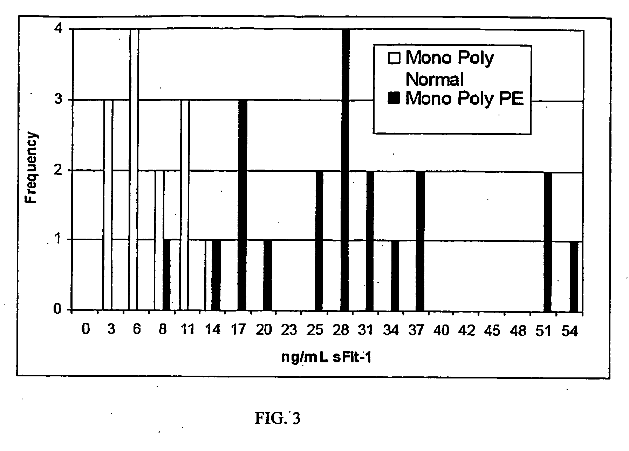

Figure 3 depicts a histogram of data collected using a monoclonal antibody as a first sbp for sFlt-1 and a polyclonal antibody as a second sbp for sFlt-1. These data show that there is a significant overlap in the range of total sFlt-1 values for non-preeclamptic pregnant women (Normal) with the range of total sFlt-1 values for preeclamptic women (PE). According to these data, there would be a need to improve the ability to discriminate between normal and preeclamptic pregnancies based on inspection of sFlt-1 levels observed by an immunoassay using two antibodies to sFlt-1 in diagnostic samples obtained from pregnant women.

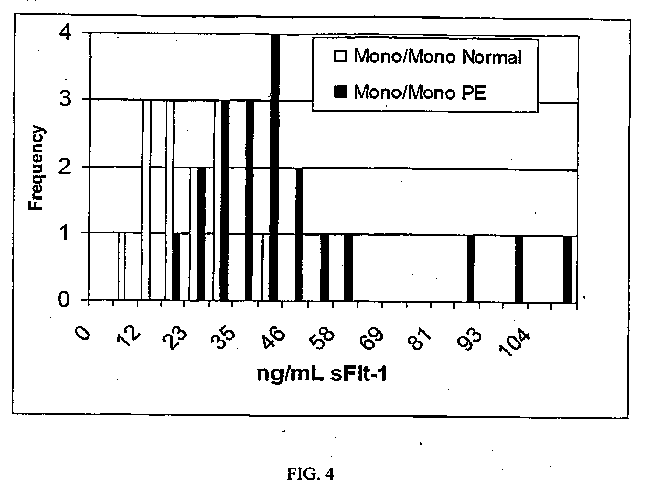

Figure 4 depicts data obtained in a manner similar to that of the data depicted in Figure 3, except that two monoclonal antibodies to sFlt-1 were used instead of a combination of a polyclonal and a monoclonal antibody. The data presented in Figures 3 and 4 indicate that an immunoassay for sFlt-1 comprising a polyclonal and monoclonal antibody for sFlt-1 outperforms a similar immunoassay comprising two monoclonal antibodies. Even though based on general principles one would expect that this assay would provide better quantitation than the assay of Figure 3, it surprisingly provide less ability to discriminate non-preeclamptic specimens from preeclamptic specimens.

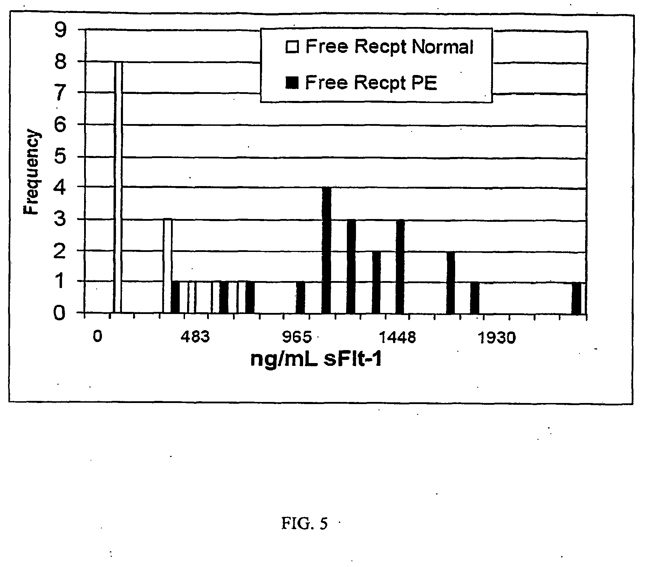

Figure 5 depicts a histogram of data collected using one preferred embodiment of the present invention. These data were collected with an immunoassay comprising a microparticle-labeled monoclonal antibody to sFlt-1 so that total sFlt-1 in the sample would be bound to the microparticle. The bound sFlt-1 was detected by sFlt-1-binding portion of P1GF labeled with acridinium. This assay determines the amount of free sFlt-1 in the specimen. These data show that there is a significant reduction in the overlap of free sFlt-1 values for non-preeclamptic pregnant women (Normal) with the range of values of free sFlt-1 for preeclamptic women (PE). According to these data, use of a portion of P1GF as a sbp for free sFlt-1 significantly improves the ability to discriminate between normal and preeclamptic pregnancies based on inspection of sFlt-1 levels in diagnostic samples obtained from pregnant women.

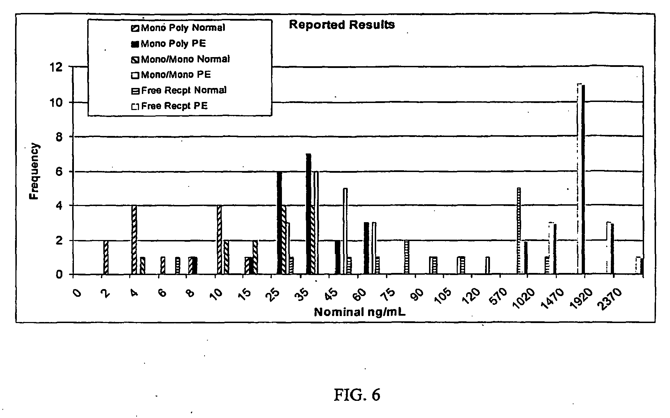

Figure 6 collects the data described above and presents it in a single graphic representation.

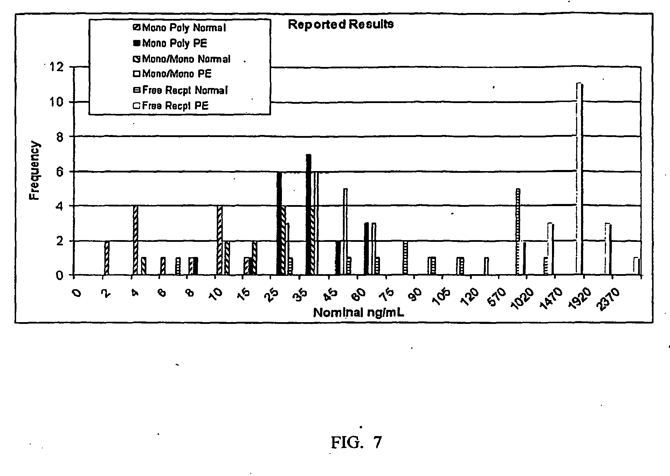

Figure 7 normalizes the data presented in Figure 6 to a single non-preeclamptic sample.

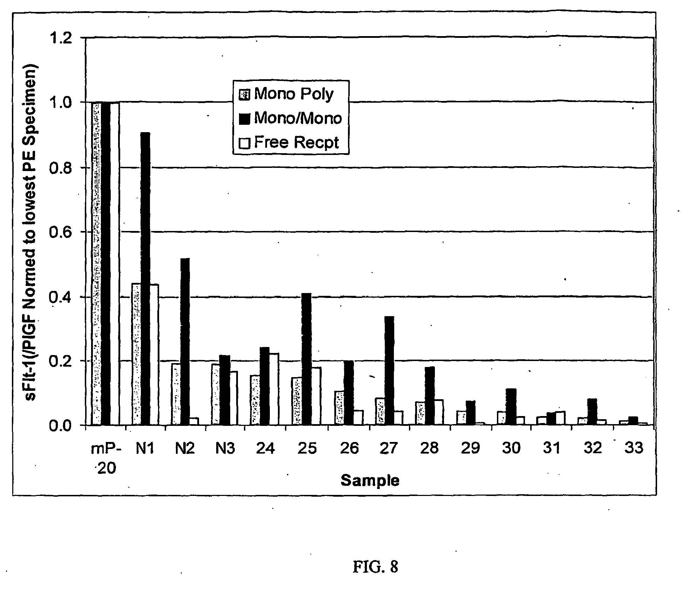

Figure 8 compares data collected from the "most normal" preeclamptic woman in the study ("mP-20") to data collected from the samples of non-preeclamptic women. Measurements of sFlt-1 are of total sFlt-1 for the monoclonal+polyclonal antibody format of this assay, and for the monoclonal+monoclonal format of this assay, whereas for the "Free Recpt" data, an sFlt-1-binding portion of P1GF was used as one sbp in a sandwich immunoassay, and therefore, bound only to free sFlt-1. These data show that, in accordance with aspects of the present invention, the ratio of free sFlt-1 to free PlGF (ranging from about 0 to about 1.0) is a better predictor of lower risk (i.e., normal pregnancies) than the ratio of P1GF to total sFlt-1 in the sample.

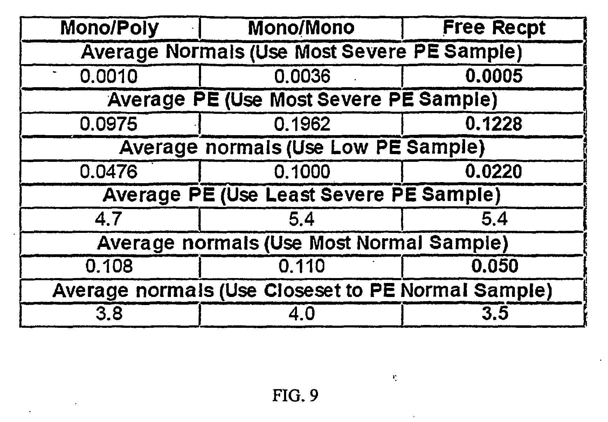

Figure 9 depicts in tabular form the increased ability of the present invention to discriminate non-preeclamptic from preeclamptic samples. These data show that for non-preeclamptic specimens determined with a two-antibody based immunoassay the ratio of free sFlt-1 to free PlGF is observed to be higher than when using embodiments of the invention. Accordingly, these data show that the invention provides superior discrimination of non-preeclamptic specimens from preeclamptic samples.

### Detailed Description of the Invention

In describing the invention, the following terms and abbreviations will be used.

### Abbreviations used to Describe the Invention

The abbreviation "sbp" refers to a "specific binding partner". All biological materials have some affinity for each other, however, specific binding partners are those that bind together in a specific way to perform a biological function. For example, sFlt-1 binds to PlGF with a biologically significant affinity, and by so doing, is able to modulate the biological activity of P1GF. Accordingly, sFlt-1 and P1GF are specific binding partners. Similarly, the membrane bound counterpart to sFlt-1, i.e., Flt-1, is also a sbp of P1GF. Moreover, VEGF is also a sbp with Flt-1. Another type of sbp that is useful in the context of the invention is an antibody and the molecule comprising the epitope to which it binds. While this type of sbp is essential to the function of some of the embodiments of the present invention, most embodiments of the invention are directed to determining the presence or quantity of one specific binding partner by detecting under assay conditions the binding to the other sbp, wherein neither the first or second sbp is an antibody or portion of an antibody.

The term "proteinaceous" is used herein to describe polypeptides, protein fragments, and proteins which are large enough to form at least one helix, sheet, or other significant (polypeptidyl) secondary structure. The term proteinaceous includes both unmodified and modified polypeptides, such as glycosylated, proteolytically cleaved, prenylated, and dimerized polypeptides and proteins. Each proteinaceous binding partner preferably comprises at least one element of secondary structure, such as a helical or sheet structure (e.g., by way of example, an α-helix or β-sheet). Accordingly, each proteinaceous binding partner preferably comprises at least 8 amino acid residues, more preferably at least 50 amino acid residues, and yet more preferably at least 100 amino acid residues. A proteinaceous binding partner can also comprise multiple polypeptide strands folded together so as to form a complex protein.

The terms "biological specimen" or "biological sample" are used interchangeably (unless expressly indicated to the contrary), and refer to any material originating from a human or other animal that contains proteinaceous molecules. The inventive method can usefully be performed on any suitable sample, including without limitation sewage, clothing, carpeting, respiratory condensates, and tissue biopsies. Preferred "biological samples" of the present invention include blood, blood plasma, blood serum, urine, feces, lymph, and saliva. When substantially solid biological samples are used, it will most commonly be preferable to extract or solubilize a portion of the sample prior to performing or continuing the inventive method.

The term "P1GF" refers to placental growth factor, and is sometimes referred to as PGF. The gene encoding P1GF is currently believed to map to gene locus 14q24-q31. P1GF encompasses all three isoforms currently known in the art and any others that are currently not well characterized to the extent that they bind with the P1GF sbp used in any particular embodiment of the invention.

The term "label" or "detectable label" means any suitable molecule allowing the direct or indirect quantitative or relative measurement of the molecule to which it is attached. Suitable labels useful in the context of the invention include solids, enzymes, enzyme substrates, enzyme inhibitors, coenzymes, enzyme precursors, apoenzymes, fluorescent substances, pigments, chemiluminescent compounds, luminescent substances, coloring substances, magnetic substances, metal particles such as gold colloids, radioactive substances, and the like. Useful enzymatic markers include without limitation dehydrogenases, oxidoreductases such as reductases and oxidases; transferases that catalyze the transfer of functional groups, such as amino, carboxyl, methyl, acyl, and phosphate groups; hydrolases that hydrolyze bonds such as ester, glycoside, ether, and peptide bonds; lyases; isomerases; ligases; and the like. Multiple enzymes can also be used in a conjugated form for detection.

Useful solid labels include but are not limited to microtiter plates, particles, microparticles and microscope slides.

When the detectable marker is an enzyme, detection of the labeled molecule also can be facilitated by enzymatic cycling. For example, when the detectable label is alkaline phosphatase, measurements can be made by observing the fluorescence or luminescence generated from a suitable substrate, such as an umbelliferone derivative. Useful umbelliferone derivatives include without limitation 4-methyl-umbellipheryl phosphate.

Other useful labels include phosphorylated phenol derivatives such as nitrophenyl phosphate, luciferin derivatives, dioxetane derivatives.

Preferred fluorescent and chemiluminescent labels useful in the context of the invention include fluorescein isothiocyanate; rhodamine derivatives such as rhodamine B isothiocyanate and tetramethyl rhodamine isothiocyanate; dancyl chloride (5-(dimethylamino)-1-naphtalenesulfonyl chloride), dancyl fluoride, fluorescamine (4-phenylspiro[furan-2(3H), 1'-(3'H)-isobenzofuran]-3,3'-dione); phycobiliproteins such as phycocyanine and physoerythrin; acridinium salts; luminol compounds such as lumiferin, luciferase and aequorin; imidazoles; oxalic acid esters; chelate compounds of rare earth elements such as europium (Eu), terbium (Tb) and samarium (Sm); and coumarin derivatives such as 7-amino-4-methylcoumarin.

Accordingly, it will be appreciated that a wide variety of detectable markers useful in the context of the present invention are available. It will also be appreciated that any suitable detection means can be used to quantify the amount of a molecule attached to a detectable label, such as but not limited to the use of electrodes, spectrophotometric measurement of color, light, or absorbance, and visual inspection.

### Specific Embodiments of the Invention

The present disclosure provides a method of determining the concentration or amount of a proteinaceous specific binding-pair present in a biological specimen. The binding-pair comprises two proteinaceous moieties (i.e., a first proteinaceous moiety and a second proteinaceous moiety) that preferably bind directly to each other and neither proteinaceous binding partner is an antibody (or fragment of an antibody). Any suitable proteinaceous binding partners, including polypeptides and proteins, can be used in the inventive method. Antibodies and portions of antibodies can be used in the inventive method, but preferably less than two antibodies or portions thereof are used.

Alternatively, when more than two antibodies are used, one of the two proteinaceous specific binding partners of the method is labeled with the detectable marker that is observed in the method. For example, when absorbance at a particular wavelength is used, the chromophore is linked to the either the first or second sbp other than through use of an antibody or portion thereof. Similarly, if a scintillation or Geiger counter is used to detect a radioactive label (e.g., ¹²⁵I), the sbp is indirectly, or more preferably directly, attached directly to the second sbp. When the first or second sbp is labeled indirectly through a means other than a first or second antibody, or portion of an antibody, then the directly labeled moiety is preferably either one having biospecific affinity for the first or second sbp (i.e., a third sbp) or is one having stringent affinity for a component of the first or second sbp. One example of an indirect label system having stringent affinity includes biotin and avidin. In this non-limiting example, the second sbp can be labeled with biotin and an avidin-like moiety (e.g., avidin, streptavidin, extravidin) can be directly labeled (with, e.g., an acridinium ester or other chemiluminescent acridinium derivative).

In a first embodiment of the inventive method, the first proteinaceous specific binding partner is labeled with a detectable label, which therefore forms a labeled moiety. The labeled moiety is contacted with the biological specimen and the degree of binding between the labeled moiety and the component of the biological specimen that is the second binding partner is determined. The degree of binding indicates either the concentration or amount of the first binding partner or the second binding partner present in the specimen. The determination of the degree of binding can be a relative value, but is preferably quantitative. This degree of binding can be determined by any suitable means, such as by causing the bound pair to agglutinate, bind to a solid support, or migrate at a differential rate through a liquid medium. Preferably, the degree of binding is determined by causing the bound pair to adhere to a solid support, separating the unbound labeled moiety from the bound label moiety and determining either the bound or unbound (or both) fraction of the labeled moiety.

The binding pair used in the invention is preferably not a pair of proteins that interact in the mammalian immune system such as the T cell receptor and major histocompatibility complex, CD40 and CD40L, or an antibody and its target.

The method optionally can be performed with a cartridge, test strip, or in a unitary package adapted to be used by a semi-automated or fully-automated immunoanalyzer. Automated diagnostic assays in the context of the invention are preferably performed in a system that delivers samples and reagents to a reaction vessel, performs incubations, and optionally washes unbound labeled moiety from the bound labeled moiety, without user intervention, once the sample and reagents are inserted into the system. Such a system optionally can be distinguished from manual or less-automated systems by the ability of the system to perform at least eight assays, preferably at least 16 assays, more preferably at least 64 assays, and most preferably at least 128 assays in a 48-hour period without user intervention after inserting the sample and the reagents into the system. The system is preferably also able to calculate the concentration or quantity of the target protein of the binding pair automatically, i.e., without the need for human calculation or input once the samples are loaded into the system.

The method also can be performed in a cartridge format or in a test strip assay. In such an assay, the assay reagents are preferably provided as a unit-dose loadable into disposable instrument and the unit-dose contains all the reagents necessary to assay to perform the method. Such a unit dose instrument for example can comprise a plastic housing comprising a disposable membrane-like structure of nylon, nitrocellulose, or other suitable material. The sample can be preprocessed or loaded directly onto a loading zone. The sample can then optionally flow across the membrane-like structure through a plurality of zones contained on the membrane. The membrane-like structure optionally further contains a detergent or lateral flow-aid and also optionally contains an absorbant to collect excess fluid and/or encourage the lateral flow across the membrane. Additionally, the inventive method can be performed with multi-pack systems in which each pack comprises sufficient reagents to perform 2, 4, 8, 10, or 12 assays, or preferably one assay.

The method can also be performed in a microfluidic device designed to analyze samples in the microliter range (e.g., less than 50 µL, preferably less than 12 µL, and optionally less than 4 µL of fluid). Such microfluidic devices can optionally contain flow aids, propulsion devices (including but not limited to expansion gels, waxes, and gases), nanovalving and the like to assist the transportation of the biological fluid or assay reagents or both through the microfluidic device.

The method optionally can be configured as a sandwich assay. Sandwich assays comprise binding the labeled moiety to the other specific binding partner of the binding pair, and another specific labeling reagent. Multiple sandwich assays are within the scope of the invention. Mainly for the sake of illustration and ease of comprehension, but not by limitation, the following sandwich assays are illustrated by the use of P1GF as the first binding partner and sFlt-1 as the second binding partner. However, any suitable pair of proteinaceous specific binding partners can be substituted for the P1GF and/or sFlt-1 in the following illustrations.

In a first sandwich assay within the scope of the invention an antibody to P1GF (α-P1GF) is bound to a microtiter plate which antibody is previously or subsequently bound to P1GF or an sFlt-1-binding fragment thereof. In the context of the invention, the PlGF is thus labeled with a solid substrate and is a labeled moiety. The microtiter plate can be optionally washed to ensure that substantially no free P1GF is on the microtiter plate. A sample is contacted to the plate, which sample is known to contain, or suspected of containing sFlt-1. Thus, the use of PlGF:sFlt-1 binding is used in the assay. After washing, the quantity of sFlt-1 in the sample can be detected by contacting the plate with a labeled antibody. The antibody can be labeled with any suitable detectable label.

In a second sandwich assay within the scope of the invention the microtiter plate of the first sandwich assay is replaced a microparticle. Preferred microparticles include but are not limited magnetic microparticles, particularly those averaging between 0.2 and 7.0 microns in size, haptenated microparticles, microparticles impregnated by one or preferably at least two fluorescent dyes (particularly those that can be identified after individual isolation in a flow cell and excitation by a laser), ferrofluids (i.e., magnetic particles less than about 0.1 µm in size), and other microparticles removable by collectable or removable by filtration.

In a third and fourth sandwich assay within the scope of the invention, the P1GF is conjugated directly to the microtiter plate or to the microparticle, respectively.

In a fifth and sixth sandwich assay, the P1GF is biotinylated or labeled with a suitable hapten, such as for example, adamantine, fluorescein isothiocyanate, or carbazole. This allows the formation of aggregates when contacted with a multi-valent antibody or (strept)avidin containing moiety, or alternatively allows easy attachment of the P1GF to a solid substrate such as a microtiter plate, microscope slide, or microparticle. In embodiments employing aggregation, any suitable separation or detection means can be used, such as precipitation or filtration of the aggregates or liquid chromatography of the aggregates.

Similarly, the inventive method comprises competitive inhibition assays. A competitive inhibition assay can be configured with a single specific binding partner or also as a sandwich assay. Useful competitive inhibition assays include those in which a labeled second specific binding partner (or fragment thereof) ("labeled 2^{nd} sbp") is synthesized or isolated from a source other than the biological sample to be assayed, and labeled with a direct or indirect label. The amount of the 2^{nd} sbp in the tested biological sample is then determined by measuring the extent to which the labeled 2^{nd} sbp is prevented from binding to the first sbp. By way of illustration, and not limitation, and using the illustrative convention used above, sFlt-1 or a PlGF-binding fragment thereof can be labeled with any suitable detectable label, including without limitation those discussed above. When immobilized P1GF is contacted with a biological sample, the sFlt-1 in the biological sample will compete with the labeled sFlt-1 for binding to the P1GF. The reduction in label binding to the immobilized P1GF then indicates the amount of sFlt-1 in the biological sample which is known to contain, or is suspected of containing, s-Flt-1.

The skilled artisan will appreciate, therefore, that the disclosure provides many embodiments in which the binding interaction of a first polypeptide or protein with a second polypeptide or protein is used to measure the amount or concentration of the second polypeptide or protein. The specific binding partners used to illustrate embodiments of the invention above, are, PlGF and sFlt-1. Additional preferred embodiments include other angiogenic growth factors and their receptors, such as without limitation, VEGF-A; VEGF-B, VEGF-C, VEGF-D, VPF and the like. Similarly, the biologically significant variants of the growth factors, such as without limitation, VEGF-A₂₀₆, VEGF-A₁₈₉, VEGF-A₁₆₅, VEGF-A₁₄₅, and VEGF-A₁₂₁ are preferred specific binding partners of the invention. Similarly, receptors for these molecules, such as KDR, or Flk-1 (fins tyrosine-like kinase-1) (also VEGFR or VEGFR2) are preferred first or second binging partners of the present invention.

It will be readily appreciated that the first specific binding partner or the second specific binding partner can be a chimera or fusion comprising amino acid residues from other polypeptides. Similarly, the specific binding partners can be full-length or truncated. Particularly preferred truncations useful in the context of the invention are those that cleave a transmembrane region from a soluble extracellular domain of the protein, although the method can also be performed using membrane-bound or bindable binding partners. When a specific binding partner is membrane bound the membrane optionally can be part of a cellular structure, synthetic, or removed from a cellular context (e.g., in a vesicle, liposome, or emulsion). This extracellular domain itself can be "full length", truncated at the N-terminus or C-terminus or both, and can be fused to exogenous polypeptides.

The invention also provides a method of determining the concentration of sFlt-1 that does not have a specific binding partner bound to the P1GF binding site of sFlt-1. The method includes contacting a sample that is known or suspected of containing sFlt-1 that does not have a specific binding partner bound to the PlGF binding site of sFlt-1 with a first specific binding partner (sbp) of sFlt-1 capable of forming a sbp:sFlt-1 complex and with a second sbp, wherein the second sbp is specifically labeled with a detectably label or a solid structure. The first or second sbp is P1GF or an sFlt-1-binding fragment of P1GF. The first sbp, the second sbp, and the sFlt-1, if present, then form a ternary complex which is detected as an indication of the amount of sFlt-1 in the sample.

In one preferred embodiment the total sFlt-1 in the biological sample is captured with an Flt-1 binding antibody and the portion of sFlt-1 in the sample that is not bound to PlGF or a similar binding partner that binds to the PlGF-binding site of sFlt-1 is detected with a labeled fragment of the epidermal growth factor (EGF) superfamily. Suitable members of the EGF superfamily include, but are not limited to, any suitable portion of P1GF or VEGF. In yet more preferred embodiments that EGF superfamily member is labeled with a luminophore or an enzyme capable of producing a detectable product, such as without limitation, horse radish peroxidase, fluorescein, or acridinium.

Another preferred embodiment comprises affixing P1GF on a solid surface, such as without limitation a microparticle. This reagent will capture only free sFlt-1. Without desiring to be bound by any particular theory it is believed that the sFlt-1 in the tested biological sample does not bind to the solid-bound P1GF because the binding site between P1GF and sFlt-1 is already bound in non-free sFlt-1. The complex can then be detected in any suitable manner. Suitable direct and indirect detection reagents include an antibody, antibody-fragment, or aptamer to the sFlt-1.

Any of the reagents in the foregoing embodiments can be readily biotinylated through prior art methods. Accordingly, the P1GF can be biotinylated which will facilitate its fixture to a solid phase or another detectable molecule, and the detection reagent can be biotinylated so that it is detectable with a specific binding partner for biotin. Preferred specific binding partners for biotin in the context of the invention include antibodies and aptamers to biotin, avidin and strepavidin.

The structure of sFlt-1 is well-known in the art (See, Wiesmann et al., Cell, 91, 695-704 (1997); Davis-Smyth et al., EMBO J, 15, 4919-4927 (1996); Barleon et al., J Biol Chem, 272, 10382-10388 (1997); Cunningham et al., Biochem Biophys Res Commun, 231, 596-599 (1997); Fuh et al. (cited within Wiesmann et al.)). A preferred sFlt-1 specific binding partner is any suitable sFlt-1-binding fragment to P1GF. Preferred sFlt-1-binding fragments of polypeptides comprising at least about 90% of the second and third domains of sFlt-1. Truncated polypeptides of P1GF are also preferred, such as the 21^{st} amino acid of P1GF through domain 3 of P1GF. Either or both of the P1GF and sFlt-1-binding fragment of P1GF can be labeled as disclosed elsewhere herein.

To facilitate detection of the interaction of the PlGF capture or detection reagent and the sFlt-1 that was free in the tested biological sample, an additional reagent can be added which is labeled by binding to a solid surface or a detectable label. Labeled antibodies are among the preferred additional reagents.

The invention also provides an immunoassay based on the competitive inhibition of a labeled sFlt-1 moiety by the quantity of sFlt-1 in the tested biological sample that does not have P1GF bound to the sFlt-1 ("free sFlt-1"). The method comprises contacting a sample that contains free sFlt-1 with a first sbp, in which the first sbp contains an sFlt-1-binding fragment of P1GF and a second sbp, in which the second sbp contains a fragment of sFlt-1 that is capable of binding to the sFlt-1 binding fragment of P1GF where at least the first sbp or the second sbp is labeled. The concentration of sFlt-1 present in the sample is then determined by measuring the decrease in binding between the first sbp and the second sbp caused by the sample.

The invention also provides a method of determining the ratio of free sFlt-1 to total sFlt-1 in a sample. The method comprises (i) determining the amount of sFlt-1 according to any of the foregoing embodiments, (ii) determining the total amount of sFlt-1 in the sample, and comparing the result of part (i) to part (ii). Any suitable method can be used to determine the total amount of sFlt-1 in the sample. Suitable methods for carrying out this step include, but are not limited to, sandwich immunoassays and competitive inhibition assays. If at least one antibody used in an immunoassay to determine the total sFlt-1 present in the assay binds to the binding site of PlGF or another factor present or possibly present in the biological sample (e.g., an anti-idiotypic antibody specific for the active site of a first or second sbp), then a portion of the sample optionally can be denatured to disrupt the binding of the sFlt-1 to other proteins in the biological sample. In this instance, any suitable technique can be used to denature the sFlt-1 such that proteins that would block the antibody binding to the active site of sFlt-1 are released. Suitable techniques include adding acid, base, salt, detergents or surfactants, organic bases or a combination of the foregoing and are within the skill of those in the art. To facilitate the binding of an antibody or another sbp used as a diagnostic reagent, the denaturant used to disrupt the binding of sFlt-1 to the sbp in the sample is preferably readily neutralized or removed from the sample. Preferably, however, the one or more antibodies used in an immunoassay to determine total sFlt-1 does not bind to the PlGF binding site of sFlt-1. The skilled artisan will appreciate that still other methods of measuring total sFlt-1 in the sample are readily available and within the scope of the present invention. Accordingly, the invention enables both the direct and indirect determination of each of (i) free sFlt-1, (ii) bound sFlt-1, and (iii) total sFlt-1. Any of these sFlt-1 values optionally can be further compared to the concentration of an EGF superfamily member, including without limitation VEGF, and preferably PlGF.

In a particularly preferred embodiment, an anti-sFlt immunoreagent is attached to a magnetic microparticle, and the biological specimen is contacted to the anti-sFlt-1 bound microparticle such that the sFlt-1 in the sample is bound to the magnetic microparticle. The complex can then be optionally washed in a suitable solution or buffer one or more times to remove unbound molecules that could interfere with the assay. Then labeled PlGF is contacted to microparticle containing complex and unbound labeled P1GF is removed or washed away from the magnetic microparticle. The amount of labeled P1GF bound to the magnetic microparticle then serves as an indication of the quantity of free sFlt-1 in the biological specimen because sFlt-1 bound by a sbp (which spb binds to the P1GF binding-site of sFlt-1) cannot efficiently bind the labeled PlGF.

In another embodiment of the claimed invention, the total sFlt-1 and the portion of the sFlt-1 bound to P1GF is measured. Any suitable method can be used to determine the quantity of total and PlGF-bound sFlt-1 ("bound sFlt-1") present in the sample. One suitable method to determine the quantity of bound sFlt-1 in the sample is to detect the formation of a complex having at least three components including an anti-PlGF antibody, the bound sFlt-1 (which itself comprises at least sFlt-1 and P1GF), and an anti-sFlt-1 antibody. That is, to employ a two-antibody sandwich assay in which one antibody is specific for P1GF and at least one antibody is specific for sFlt-1. In accordance with other preferred embodiments of the invention, the antibodies are each preferably labeled with detectable labels. In an even more preferred embodiment of this embodiment, one antibody is labeled by attachment to a solid substrate and at least one antibody is labeled by conjugation to another label referred to herein.

In another embodiment, the detection of free sFlt-1 is performed with an antibody that binds to an epitope that is not accessible (i.e., hidden) when PlGF is bound to the sFlt-1. In this way respect, the assay of the invention is any traditional sandwich, competitive inhibition, or other conventional immunoassay (for sFlt-1), except that it only measures free sFlt-1. This allows comparison of the quantity of free sFlt-1 to the quantity of total PlGF, or more preferably, to the quantity of free PlGF. In further aspects of this embodiment, an antibody to the sFlt-1 binding site of PlGF can be substituted for the portion of the sFlt-1 used in other embodiments of this invention.

The measurements of PlGF, and sFlt-1, including without limitation the measurements bound and free states of these molecules can be used for any suitable purpose. For example, the measurement of these markers can be used to predict the course of angina following a major cardiovascular event such as a non-lethal myocardial infarction. Similarly, the ability to measure these markers can be used to better understand the mode of action of heart medicines. Moreover, the accurate measurement of these markers permits more detailed investigations into the mechanisms of restenosis and neovascularization. The measurement of P1GF and sFlt-1 could find the greatest significance in demonstrating a lower risk of preeclampsia in pregnant women.

Preeclampsia affects about 5% of all pregnant women, and in some ethnic groups affects as many as about 10% of all pregnant women. The effects of preeclampsia can be severe and sometimes include death. Accordingly, there is a need to better separate normal pregnancies from pregnancies at high risk for preeclampsia.

The present inventors have discovered that the ratio of free sFlt-1 to free PlGF is a better predictor of risk of preeclampsia than is the ratio of total sFlt-1 to free PlGF. Because the quantity of free sFlt-1 is mathematically related to the quantity of total sFlt-1 and bound sFlt-1, these values can be used as a surrogate for the quantity of free sFlt-1, and can be compared to the quantity of P1GF in a biological sample within the scope of the present invention.

Many proteins of interest for medical diagnostics are present in low concentrations, e.g., at from less than 1 pg/mL to 0.1 mg/mL. Some of these proteins will bind to a protein receptor with affinities similar to that observed for antibody-antigen interactions. In an analogous fashion to enzymatic activity, it is possible to measure the amount of a free protein or the amount that is bound its native receptor.

Preeclampsia is a disease of late pregnancy that is currently diagnosed based on clinical symptoms of high blood pressure and protein in the urine. Recent literature has proposed that the precipitating event of the disease is a decrease in circulating levels of the angiogenic proteins Vascular Endothelial Growth Factor (VEGF) and Placental Growth Factor (PlGF). The resulting lack of vascularization in the placenta is then suggested to be responsible for the increase in blood pressure and proteinuria, clinically known as preeclampsia. The decrease in these two proteins is apparently due to the increased concentration of the soluble form of the receptor soluble fms-like tyrosine kinase 1(sFlt-1). The present invention covers an approach to measuring sFlt-1, which is free or bound to P1GF and its use as an assay component for measuring free and bound forms of P1GF. For detection of preeclampsia, the most relevant information is the levels of PlGF in relationship to that of the sFlt-1 which is not bound to P1GF. High concentrations of free sFlt-1 indicate that the P1GF concentrations are likely to be low due to the presence of a large excess of free receptor.

In the preferred embodiment, an antibody is used to bind all the circulating sFlt-1 (either bound or unbound to P1GF). A conjugate of a signal generating moiety and PlGF is then allowed to interact with the sFlt-1 bound to the solid phase. In this example, only the sFlt-1 free of PlGF would bind the conjugated P1GF. The unbound P1GF is then washed away and the necessary steps are taken to reveal the concentration of PlGF-conjugate.

The above format could also be constructed using VEGF, in the same manner as the P1GF as conjugate. Furthermore it would be possible to also use the heterodimer of VEGF and P1GF.

Another form of the assay would be to use P1GF bound to a solid phase and then capture any free PlGF which can then be detected with an conjugated antibody that binds sFlt-1. The same format can use VEGF on the solid phase.

Another form of the assay would measure free P1GF or VEGF by attaching the sFlt-1 to a solid phase and then capturing any free growth factor that is not bound to a soluble receptor. Detection again can be performed with a conjugated antibody that binds to the growth factor. This form of the assay would be specific for the biologically active form of the growth factors. In this format any degraded growth factor would not be detected improving the specificity of the assay to the biological event that causes preeclampsia.

When the relevant biological question is the amount of P1GF bound to receptor, it is also possible to use a solid phase that would capture sFlt-1 as in the first example and then use a conjugated antibody against P1GF or VEGF to measure the amount of bound growth factor. The utility of the approach would depend upon the successful correlation of disease state with the species measured.

Another form of the assay is to use immobilized receptor in a competitive format where the free ligand in the sample competes with labeled ligand. For example sFlt-1 on a solid phase be used to capture either the P1GF in the sample in a competitive format with labeled PlGF. This form of the assay would eliminate the requirement of an antibody in the assay.

The converse of the above example would be to immobilize with PlGF or VEGF and add sample and conjugated sFlt-1. In this format, free sFlt-1 would compete for sites on the solid phase.

The sources of the protein used in the assay could be derived from patient samples however the use of recombinant proteins expressed in either cell culture or in bacteria would be more practical approach.

The approach described here could be used to interrogate samples with regards to either receptor or associated ligand activity so long as the affinity between the ligand and the receptor is sufficiently high to permit the use of wash steps without such loss of the bound material to such an extent that it could not be detected in the signal generation of the assay protocol.

Assays that depend upon the inherent biological binding activity of the targeted proteins may provide superior information to assess the clinical situation of a patient. When a disease or medical condition involves a protein receptor, assays that measure the relative amount of that biological activity can be expected to lead to a more accurate clinical picture as compared to only knowing the mass of the protein.

In accordance with the foregoing methods, the present invention also provides an immunoassay comprising two proteinaceous specific binding partners, wherein at least one sbp is detectably labeled.

Additionally, in accordance with the foregoing the disclosure provides a composition of matter for determining the ratio of free sFlt-1 to free P1GF, as well as compositions of matter for determining the total (i) sFlt-1 and bound sFlt-1 or (ii) the total P1GF and bound P1GF, or both (i) and (ii).

### Examples

The invention is illustrated with data obtained from various immunoassays for total P1GF, free PlGF, total sFlt-1, and free sFlt-1. A selection of these data deemed to be most illustrative of the invention and inventive concepts are presented in the attached drawings and discussed briefly in the Brief Description of the Drawings. As is clear from the entirety of this description of the invention, the examples are meant to illustrate the claimed invention rather than to limit its scope.

### Further Examples

The following additional examples provide more detail regarding two preferred embodiments of the present invention.

### EXAMPLE 1

This example illustrates the inventive method in an assay used to detect free sFlt-1 in a biological sample using a portion of PlGF as a sbp for sFlt-1.

A monoclonal antibody against sFlt-1 was coated on magnetic carboxyl-latex microparticles (4.7 microns) at a protein concentration of 0.1mg/mL of microparticles at a concentration of 1% by weight in 50mM sodium MES (2-morpholinoethanesulfonic acid) at pH 6.0. After 10 minutes, EDAC, (ethyl-3-(-3-dimethylaminopropyl)carbodiimide) was added and allowed to react for one hour before washing the particles with phosphate buffered saline. The particles were then diluted to 0.1% in a buffer for use in an automated immunochemical analyzer.

Acridinylated P1GF was prepared by dissolving P1GF in phosphate buffered saline and incubation with an acridinium-ester at a mass ratio of PlGF to acridinium of 150,00/1. The conjugate was then purified by HPLC chromatography and diluted to a concentration of approximately 75 ng/mL.

The following series of steps are then performed. A 0.05 mL aliquot of sample is added to a reaction vessel to which 0.05 mL of the 0.1% labeled microparticles is added. The reaction mixture is incubated for 18 minutes at 37 degrees centigrade. A magnet holds the particles while the reaction solution is removed. After the particles are washed, a 0.05 mL aliquot of conjugate solution is added. After incubation for 4 minutes, the particles are once again held to a magnet and the pellet of microparticles the conjugate solution is removed followed by washing of the particles once again. The remaining acridinium label is caused to emit light after the addition of sodium hydroxide and hydrogen peroxide. The photons released are measured and is linearly related to the calibrators run in the identical way.

Data were collected on test samples and compared to the results obtained with conventional immunoassays. The data suggested that the inventive method better discriminated non-preeclamptic samples from preeclamptic samples, particularly when observing the ratio of free sFlt-1 to free PLGF concentrations.

### EXAMPLE 2

This example illustrates the inventive method in an assay used to detect free PlGF in a biological sample using a portion of sFlt-1 as a sbp for P1GF.

Paramagnetic latex microparticles (4.7 microns), derivatized with carboxyl functional groups, was coated with anti-sFlt-1 antibody (containing domains 1-3 of the fms-like tyrosine kinase 1) at a protein concentration demonstrated to be sufficient to maximize the amount of protein absorbed to the surface area of the microparticles (2% solids by weight) in 50mM MES, pH 5.5. In another embodiment, the sFlt-1 could be bound directly to a solid substrate. After 10 minutes, the non-absorbed sFlt-1 was removed by washing the particles multiple times with MES buffer. Following washing the particles, EDAC was added and allowed to react forming a covalent coupling of the sFlt-1 molecules to the particles. The particles were then washed with phosphate buffered saline to stop the reaction and remove unreacted EDAC. The particles are then diluted to 0.1 % in buffer for use in an automated immunochemical analyzer.

Acridinium-labeled anti-P1GF antibody was prepared by incubating an polyclonal antibody (alternatively a monoclonal antibody could be used) with an acridinium-ester at a molar ratio of acridinium to antibody ranging from 1 to 100. Unconjugated acridinium was then separated from the acridinium-labeled antibody conjugate by size chromatography. The purified conjugate was then diluted in buffer to a concentration yielding the maximum signal to noise ratio in the assay.

The following series of steps were then performed. A 0.1 mL aliquot of sample was added to a reaction vessel to which 0.05 mL of the 0.1% labeled microparticles was added. The reaction mixture was incubated for 18 minutes at 37 degrees centigrade. Utilizing the paramagnetic property of the particles, a magnet attracts and holds the particles against the side of the reaction vessel while the reaction solution is removed. After the particles are washed, buffer is dispensed; a 0.05 mL aliquot of conjugate solution is added; the magnet removed and the mixture vortexed. After a 4-minute incubation, excess conjugate was removed by particle attraction to a magnet, washing and resuspension. The particles, now containing the sFlt-1/PlGF/anti-PlGF antibody (acridinium-labeled) sandwich, was then exposed to reactants causing the acridinium to emit light. The chemiluminescence, measured by the instrument, is directly proportional to the amount of P1GF (free) in the sample.

Data were collected on test samples and compared to the results obtained with conventional immunoassays. The data suggested that the inventive method better distinguished the preeclamptic state from the non-preeclamptic state by measuring the biological activity of the prophylactic or causative biological agent rather than using antibodies against the agent that would not necessarily distinguish between active and inactive forms of the protein.

## Claims

1. A method of predicting a lower risk of preeclampsia comprising: measuring the amount of free sFlt-1 in a biological sample obtained from a pregnant woman, measuring the amount of free P1GF in the biological sample, and comparing the observed ratio to a predetermined value or range of values.

2. A method of predicting a lower risk of preeclampsia comprising: measuring the amounts of total sFlt-1 and bound sFlt-1 in a biological sample obtained from a pregnant woman, measuring the amount of free P1GF in the biological sample, and comparing the observed ratio to a predetermined value or range of values.

3. A method of diagnosing preeclampsia comprising :
(a) determining the amount of free sFlt-1 in a sample,
(b) determining the amount of free P1GF in a sample, and
(c) dividing the value of step (a) by the value of step (b),
wherein when the result of step (c) exceeds a predetermined value then it is diagnostic of preeclampsia.

4. The method of claim 1 or 2 wherein the amount of free P1GF is determined by the steps of:
(a) providing a labeled moiety, wherein the labeled moiety is a fragment of PlGF or fragment of sFlt-1,
(b) contacting the labeled moiety with the biological specimen,
(c) when the labeled moiety comprises P1GF, determining degree of binding between the labeled moiety and sFlt-1, or
(c') when the labeled moiety comprises sFlt-1, determining the degree of binding between the labeled moiety and P1GF, and
(d) determining the concentration or amount of free-PlGF present in the sample.

5. The method of claim 4, wherein the method is an automated diagnostic assay, wherein the automated assay is performed in a system that delivers samples and reagents to a reaction vessel, performs incubations and (optionally washes) without user intervention once the sample and reagents are inserted into the system.

6. The method of claim 5, wherein the system can perform at least eight assays in a 48-hour period, without user intervention after inserting the sample and the reagents into the system.

7. The method of claim 5 or 6, wherein the system calculates the concentration or quantity of the protein of the binding pair and wherein the user is not considered to be a part of the system.

8. The method of claim 3, wherein the method is a sandwich assay.

9. The method of claim 3, wherein the method is a competitive inhibition assay.

10. The method of claim 1 or 2 wherein the amount of free P1GF in the_biological sample, wherein free P1GF does not have a specific binding partner bound to the FIt-I binding site of PlGF, is determined by the steps of:
(a) providing a sample that contains or is suspected of containing free PlGF,
(b) contacting the sample with a first specific binding partner (sbp) of P1GF capable of forming a sbp:PlGF complex,
(c) contacting the sample with a second sbp, wherein the second sbp is specifically labeled, wherein either the first sbp or second sbp is Flt-I or sFlt-1 or a PlGF-binding fragment of sFlt-1, wherein the first sbp, the second sbp, and the free P1GF are capable of forming a ternary complex,
(d) determining the amount of ternary complex formed as a measure of the amount of free PlGF in the sample.

11. The method of claim 2 wherein the amount of total sFlt-1 is determined by the steps of:
(a) labeling a binding partner with a detectable label to form a labeled moiety,
(b) contacting the labeled moiety with the biological specimen, and
(c) determining the degree of binding between the labeled moiety and a component of the biological specimen as an indication of the amount of sFlt-1 present in the specimen.

12. The method of claim 11,
wherein the method is performed in a cartridge format or as a test strip, or
wherein the assay reagents are provided as a unit-dose disposable instrument,
and wherein the unit-dose contains all the reagents necessary to assay perform the method.

13. The method of claim 11 or 12, wherein the method is an automated diagnostic assay, wherein the assay is performed in a system that delivers samples and reagents to a reaction vessel, performs incubations (and optionally washes) without user intervention once the sample and reagents are inserted into the system.

14. The method of claim 13, wherein the system can perform at least eight assays in a 48-hour period, without user intervention after inserting the sample and the reagents into the system.

15. The method of claim 13 or 14, wherein the system calculates the concentration or quantity of sFlt-1.

16. The method of any of claims 11-15, wherein the method is a sandwich assay.

17. The method of any of claims 11-15, wherein the method is a competitive inhibition assay.

18. The method of any of claims 11-17, wherein the method employs less than two antibodies or portions of antibodies.

19. The method of any of claims 11-18, wherein the P1GF is cell-bound.

20. The method of claim 1 wherein the amount of free sFlt-1 in the biological sample, is determined by the steps of:
(a) providing a sample that contains or is suspected of containing free sFlt-1,
(b) contacting the sample with a first specific binding partner (sbp) of sFlt-1 capable of forming a sbp:sFIt-1 complex,
(c) contacting the sample with a second sbp,
wherein the second sbp is detectably labeled,
wherein either the first sbp or second sbp is PlGF, VEGF, or an sFlt-1-binding fragment of P1GF or VEGF,
wherein the first sbp, the second sbp, and the sFlt-1 are capable of forming a ternary complex,
(d) determining the amount of ternary complex formed as a measure of the amount of sFlt-1 in the sample.

21. The method of claim 1 wherein the amount of free sFlt-1 in the biological sample is determined by the steps of:
(a) providing a sample that contains or is suspected of containing free sFlt-1,
(b) contacting the sample with a solid-phase-attached antibody that specifically binds sFlt-1, whereby all the sFlt-1 in the biological sample is captured to the solid,
(c) contacting the sample with a detectably labeled portion of a member of the PlGF/VEGF family, and
(d) determining the amount of ternary complex formed as a measure of the amount of sFlt-1 in the sample.

22. The method of claim 1 wherein the amount of free sFlt-1 in the biological sample_is determined by the steps of:
(a) providing a sample that contains or is suspected of containing free sFlt-1,
(b) contacting the sample with a portion of P1GF or VEGF immobilized on a solid surface,
(c) contacting the sample with a detectably labeled antibody that specifically binds sFlt-1,
wherein the sFlt-1, detectably labeled antibody and P1GF or VEGF are capable of forming a ternary complex, and
(d) determining the amount of ternary complex formed as a measure of the amount of sFlt-1 in the sample.

23. The method of claim 22, wherein the portion of PlGF or VEGF is a portion of PlGF, and the sFlt-1-binding portion of P1GF lacks the first 21 amino acids of PlGF.

24. The method of claim 23, wherein the portion of P1GF further comprises all of domain 1 other than about the first 20 amino acid residues of domain 1.

25. The method of claim 20, wherein the P1GF or sFlt-1-binding fragment of PlGF is detectably labeled by a label other than a solid surface.

26. The method of claim 25, wherein the label is an acridinium derivative.

27. The method of claim 20, wherein either the first sbp or second sbp is an antibody to sFlt-1.

28. The method of claim 1 wherein the amount of free sFlt-1 in the biological sample is determined by the steps of:
(a) providing a sample that contains or is suspected of containing free sFlt-1 that does not have PlGF bound to the sFlt-1,
(b) contacting the sample with
a first sbp comprising an sFlt-1-binding portion of PlGF or VEGF and
a second sbp comprising a portion of sFlt-1 that is capable of binding to the sFlt-1 binding fragment of PlGF or VEGF,
wherein at least the first sbp or the second sbp is labeled,
(c) determining the concentration of sFlt-1 present in the sample by determining the decrease in binding, or inhibition of binding, between the first sbp and the second sbp caused by the sample relative to
the level of binding between the first sbp and the second sbp when contacted with a sample lacking free sFlt-1.

29. The method of claim 1 wherein the amount of free sFlt-1 in the biological sample is determined by the steps of:
(a) providing_a sample that contains or is suspected of containing free sFlt-1,
(b) providing a first specific binding partner (sbp) of sFlt-1 capable of forming a first sbp:sFlt-1 complex, wherein the first sbp is attached to a magnetic microparticle,
(c) contacting the sample to the first sbp bound microparticle,
(d) optionally washing the magnetic microparticle to separate unbound portions of the sample from the microparticle,
(e) contacting the sample with a second sbp,
wherein the second sbp is detectably labeled PlGF,
wherein the first sbp, the second sbp, and the sFlt-1 are capable of forming a ternary complex,
(f) washing unbound labeled P1GF away from the magnetic microparticle, and
(g) determining the amount of ternary complex formed by detecting the amount of labeled PlGF bound to the magnetic microparticle as an indication of the quantity of free sFlt-1 in the biological specimen.

## Patentansprüche

1. Ein Verfahren zur Prognose eines geringeren Risikos von Präeklampsie, das Folgendes umfasst: Messung der Menge von freier sFlt-1 in einer biologischen Probe, die von einer schwangeren Frau gewonnen wurde, Messung der Menge von freiem PlGF in der biologischen Probe und Vergleich des beobachteten Verhältnisses mit einem vordefinierten Wert oder Wertebereich.

2. Ein Verfahren zur Prognose eines geringeren Risikos von Präeklampsie, das Folgendes umfasst: Messung der Mengen von Gesamt-sFlt-1 und gebundener sFlt-1 in einer biologischen Probe, die von einer schwangeren Frau gewonnen wurde, Messung der Menge von freiem PlGF in der biologischen Probe und Vergleich des beobachteten Verhältnisses mit einem vordefinierten Wert oder Wertebereich.

3. Ein Verfahren zur Diagnose von Präeklampsie, das Folgendes umfasst:
(a) Bestimmung der Menge von freier sFlt-1 in einer Probe,
(b) Bestimmung der Menge von freiem PlGF in einer Probe, und
(c) Dividieren des Werts in Schritt (a) durch den Wert in Schritt (b),
worin, wenn das Ergebnis von Schritt (c) einen vordefinierten Wert überschreitet, es Präeklampsie anzeigt.

4. Das Verfahren gemäß Anspruch 1 oder 2, worin die Menge an freiem PlGF durch folgende Schritte bestimmt wird:
(a) Bereitstellung eines markierten Anteils, worin der markierte Anteil ein Fragment von PlGF oder ein Fragment von sFlt-1 ist,
(b) In-Kontakt-Bringen des markierten Anteils mit der biologischen Probe,
(c) wenn der markierte Anteil PlGF umfasst, Bestimmung des Bindungsgrads zwischen dem markierten Anteil und sFlt-1, oder (c') wenn der markierte Anteil sFlt-1 umfasst, Bestimmung des Bindungsgrads zwischen dem markierten Anteil und PlGF, und
(d) Bestimmung der Konzentration oder Menge an freiem PlGF, das in der Probe vorhanden ist.

5. Das Verfahren gemäß Anspruch 4, worin das Verfahren ein automatisierter Diagnosetest ist, worin der automatisierte Test in einem System durchgeführt wird, das ohne Eingreifen durch einen Benutzer Proben und Reagenzien in ein Reaktionsgefäß gibt, Inkubationen durchführt (und wahlweise wäscht), sobald die Probe und die Reagenzien in das System eingeführt wurden.

6. Das Verfahren gemäß Anspruch 5, worin das System mindestens acht Tests in einem Zeitraum von 48 Stunden durchführen kann, ohne Eingreifen des Benutzers, nachdem er die Probe und die Reagenzien in das System gegeben hat.

7. Das Verfahren gemäß Anspruch 5 oder 6, worin das System die Konzentration oder Menge des Proteins des Bindungspaars berechnet und worin der Benutzer nicht als Teil des Systems betrachtet wird.

8. Das Verfahren gemäß Anspruch 3, worin das Verfahren ein Sandwich-Test ist.

9. Das Verfahren gemäß Anspruch 3, worin das Verfahren ein Konkurrenzhemmungs-Test ist.

10. Das Verfahren gemäß Anspruch 1 oder 2, worin die Menge an freiem PlGF in der biologischen Probe, worin freies PlGF keinen spezifischen Bindungspartner hat, der an die Flt-I-Bindungsstelle von PlGF gebunden ist, durch folgende Schritte bestimmt wird:
(a) Bereitstellung einer Probe, die freies PlGF enthält oder von der dies vermutet wird,
(b) In-Kontakt-Bringen der Probe mit einem ersten spezifischen Bindungspartner (sbp) von PlGF, der in der Lage ist, einen sbp:PlGF-Komplex zu bilden,
(c) In-Kontakt-Bringen der Probe mit einem zweiten sbp, worin der zweite sbp spezifisch markiert ist, worin entweder der erste sbp oder der zweite sbp Flt-I oder sFlt-1 oder ein PlGFbindendes Fragment von sFlt-1 ist, worin der erste sbp, der zweite sbp und das freie PlGF in der Lage sind, einen ternären Komplex zu bilden,
(d) Bestimmung der Menge an ternärem Komplex, der sich gebildet hat, als Maß für die Menge an freiem PlGF in der Probe.

11. Das Verfahren gemäß Anspruch 2, worin die Menge an Gesamt-sFlt-1 durch folgende Schritte bestimmt wird:
(a) Markierung eines Bindungspartners mit einem nachweisbaren Label, um einen markierten Anteil zu bilden,
(b) In-Kontakt-Bringen des markierten Anteils mit der biologischen Probe und
(c) Bestimmung des Bindungsgrads zwischen dem markierten Anteil und einer Komponente der biologischen Probe als Indikation der Menge an sFlt-1, die in der Probe vorhanden ist.

12. Das Verfahren gemäß Anspruch 11,
worin das Verfahren in einem Patronenformat oder als Stabprobe durchgeführt wird oder
worin die Testreagenzien als Einzeldosis-Wegwerfinstrument bereitgestellt werden,
und worin die Einzeldosis alle Reagenzien enthält, die notwendig sind, damit der Test durch das Verfahren durchgeführt wird.

13. Das Verfahren gemäß Anspruch 11 oder 12, worin das Verfahren ein automatisierter Diagnosetest ist, worin der Test in einem System durchgeführt wird, das ohne Eingreifen durch einen Benutzer Proben und Reagenzien in ein Reaktionsgefäß gibt, Inkubationen durchführt (und wahlweise wäscht), sobald die Probe und die Reagenzien in das System eingeführt wurden.

14. Das Verfahren gemäß Anspruch 13, worin das System mindestens acht Tests in einem Zeitraum von 48 Stunden ohne Eingreifen eines Benutzers durchführen kann, nachdem die Probe und die Reagenzien in das System gegeben wurden.

15. Das Verfahren gemäß Anspruch 13 oder 14, worin das System die Konzentration oder Menge von sFlt-1 berechnet.

16. Das Verfahren gemäß einem beliebigen der Ansprüche 11-15, worin das Verfahren ein Sandwich-Test ist.

17. Das Verfahren gemäß einem beliebigen der Ansprüche 11-15, worin das Verfahren ein Konkurrenzhemmungs-Test ist.

18. Das Verfahren gemäß einem beliebigen der Ansprüche 11-17, worin das Verfahren weniger als zwei Antikörper oder Abschnitte von Antikörpern verwendet.

19. Das Verfahren gemäß einem beliebigen der Ansprüche 11-18, worin der PlGF zellgebunden ist.

20. Das Verfahren gemäß Anspruch 1, worin die Menge an freiem sFlt-1 in der biologischen Probe durch folgende Schritte bestimmt wird:
(a) Bereitstellung einer Probe, die freie sFlt-1 enthält oder von der dies vermutet wird,
(b) In-Kontakt-Bringen der Probe mit einem ersten spezifischen Bindungspartner (sbp) von sFlt-1, der in der Lage ist, einen sbp:sFlt-1-Komplex zu bilden,
(c) In-Kontakt-Bringen der Probe mit einem zweiten sbp, worin der zweite sbp nachweisbar markiert ist,
worin entweder der erste sbp oder der zweite sbp PlGF, VEGF oder ein sFlt-1-bindendes Fragment von PlGF oder VEGF ist,
worin der erste sbp, der zweite sbp und die sFlt-1 in der Lage sind, einen ternären Komplex zu bilden,
(d) Bestimmung der Menge an ternärem Komplex, der gebildet wurde, als Maß für die Menge an sFlt-1 in der Probe.

21. Das Verfahren gemäß Anspruch 1, worin die Menge an freier sFlt-1 in der biologischen Probe durch folgende Schritte bestimmt wird:
(a) Bereitstellung einer Probe, die freie sFlt-1 enthält oder von der dies vermutet wird,
(b) In-Kontakt-Bringen der Probe mit einem an eine feste Phase gebundenen Antikörper, der spezifisch sFlt-1 bindet, wodurch die gesamte sFlt-1 in der biologischen Probe an den Feststoff gebunden wird,
(c) In-Kontakt-Bringen der Probe mit einem nachweisbar markierten Abschnitt eines Mitglieds der PlGF/VEGF-Familie, und
(d) Bestimmung der Menge an ternärem Komplex, der gebildet wurde, als Maß für die Menge an sFlt-1 in der Probe.

22. Das Verfahren gemäß Anspruch 1, worin die Menge an freier sFlt-1 in der biologischen Probe durch folgende Schritte bestimmt wird:
(a) Bereitstellung einer Probe, die freie sFlt-1 enthält oder von der dies vermutet wird,
(b) In-Kontakt-Bringen der Probe mit einem Abschnitt von PlGF oder VEGF, der auf einer festen Oberfläche immobilisiert ist,
(c) In-Kontakt-Bringen der Probe mit einem nachweisbar markierten Antikörper, der spezifisch sFlt-1 bindet,
worin die sFlt-1, der nachweisbar markierte Antikörper und PlGF oder VEGF in der Lage sind, einen ternären Komplex zu bilden, und
(d) Bestimmung der Menge an ternärem Komplex, der gebildet wurde, als Maß für die Menge an sFlt-1 in der Probe.

23. Das Verfahren gemäß Anspruch 22, worin der Abschnitt von PlGF oder VEGF ein Abschnitt von PlGF ist und dem sFlt-1 bindenden Abschnitt von PlGF die ersten 21 Aminosäuren von PlGF fehlen.

24. Das Verfahren gemäß Anspruch 23, worin der Abschnitt von PlGF weiter die gesamte Domäne 1 außer ungefähr den ersten 20 Aminosäureresten der Domäne 1 umfasst.

25. Das Verfahren gemäß Anspruch 20, worin das PlGF oder sFlt-1 bindende Fragment von PlGF nachweisbar mit einem anderen Label als einer festen Oberfläche markiert wird.

26. Das Verfahren gemäß Anspruch 25, worin das Label ein Acridinium-Derivat ist.

27. Das Verfahren gemäß Anspruch 20, worin entweder der erste sbp oder der zweite sbp ein Antikörper für sFlt-1 ist.

28. Das Verfahren gemäß Anspruch 1, worin die Menge an freier sFlt-1 in der biologischen Probe durch folgende Schritte bestimmt wird:
(a) Bereitstellung einer Probe, die freie sFlt-1 enthält oder von der dies vermutet wird, die kein an die sFlt-1 gebundenes PlGF hat,
(b) In-Kontakt-Bringen der Probe mit
einem ersten sbp, der einen sFlt-1 bindenden Abschnitt von PlGF oder VEGF umfasst, und
einem zweiten sbp, der einen Abschnitt von sFlt-1 umfasst, der in der Lage ist, sich an das sFlt-1 bindende Fragment von PlGF oder VEGF zu binden,
worin mindestens der erste sbp oder der zweite sbp markiert ist,
(c) Bestimmung der Konzentration von sFlt-l, die in der Probe vorhanden ist, durch Bestimmung der Abnahme der Bindung, oder Hemmung der Bindung, zwischen dem ersten sbp und dem zweiten sbp, verursacht durch die Probe, im Verhältnis zu dem Grad der Bindung zwischen dem ersten sbp und dem zweiten sbp, wenn sie mit einer Probe in Kontakt gebracht werden, der freie sFlt-1 fehlt.

29. Das Verfahren gemäß Anspruch 1, worin die Menge an freier sFlt-1 in der biologischen Probe durch folgende Schritte bestimmt wird:
(a) Bereitstellung einer Probe, die freie sFlt-1 enthält oder von der dies vermutet wird,
(b) Bereitstellung eines ersten spezifischen Bindungspartners (sbp) von sFlt-1, der in der Lage ist, einen ersten sbp:sFlt-1-Komplex zu bilden, worin der erste sbp an einen magnetischen Mikropartikel gebunden ist,
(c) In-Kontakt-Bringen der Probe mit dem ersten sbpgebundenen Mikropartikel,
(d) optionales Waschen des magnetischen Mikropartikels, um ungebundene Abschnitte der Probe vom Mikropartikel zu trennen,
(e) In-Kontakt-Bringen der Probe mit einem zweiten sbp, worin der zweite sbp ein nachweisbar markierter PlGF ist, worin der erste sbp, der zweite sbp und die sFlt-1 in der Lage sind, einen ternären Komplex zu bilden,
(f) Abwaschen von ungebundenem markiertem PlGF vom magnetischen Mikropartikel, und
(g) Bestimmung der Menge an ternärem Komplex, der gebildet wurde, durch Erfassung der Menge an markiertem PlGF, der an den magnetischen Mikropartikel gebunden ist, als Indikation der Menge an freier sFlt-1 in der biologischen Probe.

## Revendications

1. Procédé de prédiction d'un plus faible risque de pré-éclampsie comprenant : la mesure de la quantité de sFlt-1 libre dans un échantillon biologique obtenu auprès d'une femme enceinte, la mesure de la quantité de PlGF libre dans l'échantillon biologique et la comparaison du rapport observé avec une valeur ou une plage de valeurs prédéterminée.

2. Procédé de prédiction d'un plus faible risque de pré-éclampsie comprenant : la mesure des quantités de sFlt-1 totale et de sFlt-1 liée dans un échantillon biologique obtenu auprès d'une femme enceinte, la mesure de la quantité de PlGF libre dans l'échantillon biologique et la comparaison du rapport observé avec une valeur ou une plage de valeurs prédéterminée.

3. Procédé de diagnostic de la pré-éclampsie comprenant:
(a) la détermination de la quantité de sFlt-1 libre dans un échantillon,
(b) la détermination de la quantité de PlGF libre dans un échantillon et
(c) la division de la valeur de l'étape (a) par la valeur de l'étape (b),
où, quand le résultat de l'étape (c) dépasse une valeur prédéterminée, il y a un diagnostic de pré-éclampsie.

4. Procédé selon la revendication 1 ou 2 où la quantité de PlGF libre est déterminée par les étapes de :
(a) fourniture d'une entité marquée, où l'entité marquée est un fragment de PlGF ou un fragment de sFlt-1,
(b) mise en contact de l'entité marquée avec le spécimen biologique,
(c) quand l'entité marquée comprend PlGF, détermination du degré de liaison entre l'entité marquée et sFlt-1, ou
(c') quand l'entité marquée comprend sFlt-1, détermination du degré de liaison entre l'entité marquée et PlGF, et
(d) détermination de la concentration ou de la quantité de PlGF libre présent dans l'échantillon.

5. Procédé selon la revendication 4, où le procédé est un test diagnostique automatisé, où le test automatisé est mis en oeuvre dans un système qui délivre des échantillons et des réactifs à un récipient réactionnel, réalise des incubations et (éventuellement des lavages) sans l'intervention de l'utilisateur une fois que l'échantillon et les réactifs sont insérés dans le système.

6. Procédé selon la revendication 5, où le système peut réaliser au moins huit tests dans une période de 48 heures, sans l'intervention de l'utilisateur après insertion de l'échantillon et des réactifs dans le système.

7. Procédé selon la revendication 5 ou 6, où le système calcule la concentration ou la quantité de la protéine de la paire liante et où l'utilisateur n'est pas considéré comme faisant partie du système.

8. Procédé selon la revendication 3, où le procédé est un test sandwich.

9. Procédé selon la revendication 3, où le procédé est un test à inhibition compétitive.

10. Procédé selon la revendication 1 ou 2 où la quantité de PlGF libre dans l'échantillon biologique, où le PlGF libre n'a pas un partenaire liant spécifique lié au site liant Flt-I de PlGF, est déterminée par les étapes de :
(a) fourniture d'un échantillon qui contient ou est suspecté de contenir PlGF libre,
(b) mise en contact de l'échantillon avec un premier partenaire liant spécifique (pls) de PlGF capable de former un complexe pls : PlGF,
(c) mise en contact de l'échantillon avec un second pls, où le second pls est marqué spécifiquement, où le premier pls ou le second pls est Flt-1 ou sFlt-1 ou un fragment liant PlGF de sFlt-1, où le premier pls, le second pls et le PlGF libre sont capables de former un complexe ternaire,
(d) détermination de la quantité de complexe ternaire formée comme mesure de la quantité de PlGF libre dans l'échantillon.

11. Procédé selon la revendication 2 où la quantité de sFlt-1 totale est déterminée par les étapes de :
(a) marquage d'un partenaire liant avec un marqueur détectable pour former une entité marquée,
(b) mise en contact de l'entité marquée avec le spécimen biologique, et
(c) détermination du degré de liaison entre l'entité marquée et un composant du spécimen biologique comme indication de la quantité de sFlt-1 présente dans le spécimen.

12. Procédé selon la revendication 11, où le procédé est mis en oeuvre dans un format de cartouche ou sous forme d'une bandelette réactive, ou bien où les réactifs du test sont fournis sous forme d'un instrument jetable à dose unitaire,
et où la dose unitaire contient tous les réactifs nécessaires pour réaliser le procédé avec le test.

13. Procédé selon la revendication 11 ou 12, où le procédé est un test diagnostique automatisé, où le test est réalisé dans un système qui délivre des échantillons et des réactifs à un récipient réactionnel, réalise des incubations (et éventuellement des lavages) sans l'intervention de l'utilisateur une fois que l'échantillon et les réactifs sont insérés dans le système.

14. Procédé selon la revendication 13, où le système peut réaliser au moins huit tests dans une période de 48 heures sans l'intervention de l'utilisateur après insertion de l'échantillon et des réactifs dans le système.

15. Procédé selon la revendication 13 ou 14, où le système calcule la concentration ou la quantité de sFlt-1.

16. Procédé selon l'une quelconque des revendications 11-15, où le procédé est un test sandwich.

17. Procédé selon l'une quelconque des revendications 11-15, où le procédé est un test à inhibition compétitive.

18. Procédé selon l'une quelconque des revendications 11-17, où le procédé emploie moins de deux anticorps ou parties d'anticorps.

19. Procédé selon l'une quelconque des revendications 11-18, où le PlGF est lié à des cellules.

20. Procédé selon la revendication 1, où la quantité de sFlt-1 libre dans l'échantillon biologique est déterminée par les étapes de :
(a) fourniture d'un échantillon qui contient ou est suspecté de contenir sFlt-1 libre,
(b) mise en contact de l'échantillon avec un premier partenaire liant spécifique (pls) de sFlt-1 capable de former un complexe pls: sFlt-1,
(c) mise en contact de l'échantillon avec un second pls,
où le second pls est marqué de manière détectable,
où le premier pls ou le second pls est PlGF, VEGF ou un fragment liant sFlt-1 de PlGF ou VEGF,
où le premier pls, le second pls et la sFlt-1 sont capables de former un complexe ternaire,
(d) détermination de la quantité de complexe ternaire formé comme mesure de la quantité de sFlt-1 dans l'échantillon.

21. Procédé selon la revendication 1 où la quantité de sFlt-1 libre dans l'échantillon biologique est déterminée par les étapes de :
(a) fourniture d'un échantillon qui contient ou est suspecté de contenir sFlt-1 libre,
(b) mise en contact de l'échantillon avec un anticorps fixé à une phase solide qui lie spécifiquement sFlt-1, de sorte que toute la sFlt-1 dans l'échantillon biologique est capturée sur le solide,
(c) mise en contact de l'échantillon avec une partie marquée de manière détectable d'un membre de la famille de PlGF/VEGF, et
(d) détermination de la quantité de complexe ternaire formé comme mesure de la quantité de sFlt-1 dans l'échantillon.

22. Procédé selon la revendication 1 où la quantité de sFlt-1 libre dans l'échantillon biologique est déterminée par les étapes de :
(a) fourniture d'un échantillon qui contient ou est suspecté de contenir sFlt-1 libre,
(b) mise en contact de l'échantillon avec une partie de PlGF ou VEGF immobilisée sur une surface solide,
(c) mise en contact de l'échantillon avec un anticorps marqué de manière détectable qui lie spécifiquement sFlt-1,
où la sFlt-1, l'anticorps marqué de manière détectable et PlGF ou VEGF sont capables de former un complexe ternaire, et
(d) détermination de la quantité de complexe ternaire formé comme mesure de la quantité de sFlt-1 dans l'échantillon.

23. Procédé selon la revendication 22, où la partie de PlGF ou VEGF est une partie de PlGF, et la partie liant sFlt-1 de PlGF est dépourvue des 21 premiers aminoacides de PlGF.

24. Procédé selon la revendication 23, où la partie de PlGF comprend en outre la totalité du domaine 1 sauf environ les 20 premiers résidus d'aminoacides du domaine 1.

25. Procédé selon la revendication 20, où le fragment liant PlGF ou sFlt-1 de PlGF est marqué de manière détectable par un marqueur différent d'une surface solide.

26. Procédé selon la revendication 25, où le marqueur est un dérivé d'acridinium.

27. Procédé selon la revendication 20, où le premier pls ou le second pls est un anticorps dirigé contre sFlt-1.

28. Procédé selon la revendication 1 où la quantité de sFlt-1 libre dans l'échantillon biologique est déterminée par les étapes de :
(a) fourniture d'un échantillon qui contient ou est suspecté de contenir sFlt-1 libre qui n'a pas PlGF lié à sFlt-1,
(b) mise en contact de l'échantillon avec
un premier pls comprenant une partie liant sFlt-1 de PlGF ou VEGF et
un second pls comprenant une partie de sFlt-1 qui est capable de se lier au fragment liant sFlt-1 de PlGF ou VEGF,
où au moins le premier pls ou le second pls est marqué,
(c) détermination de la concentration de sFlt-1 présente dans l'échantillon par détermination de la diminution de la liaison, ou de l'inhibition de la liaison, entre le premier pls et le second pls causée par l'échantillon par rapport au niveau de liaison entre le premier pls et le second pls quand ils sont mis en contact avec un échantillon dépourvu de sFlt-1 libre.

29. Procédé selon la revendication 1 où la quantité de sFlt-1 libre dans l'échantillon biologique est déterminée par les étapes de :
(a) fourniture d'un échantillon qui contient ou est suspecté de contenir sFlt-1 libre,
(b) fourniture d'un premier partenaire liant spécifique (pls) de sFlt-1 capable de former un premier complexe pls: sFlt-1, où le premier pls est fixé à une microparticule magnétique,
(c) mise en contact de l'échantillon avec la microparticule liée au premier pls,
(d) éventuellement lavage de la microparticule magnétique pour séparer les parties non liées de l'échantillon d'avec la microparticule,
(e) mise en contact de l'échantillon avec un second pls,
où le second pls est PlGF marqué de manière détectable,
où le premier pls, le second pls et la sFlt-1 sont capables de former un complexe ternaire,
(f) élimination par lavage du PlGF marqué non lié de la microparticule magnétique, et
(g) détermination de la quantité de complexe ternaire formé par détection de la quantité de PlGF marqué lié à la microparticule magnétique comme indication de la quantité de sFlt-1 libre dans le spécimen biologique.
